# EUROPEAN PATENT SPECIFICATION

(11) **EP 0 517 200 B1**
(45) Date of publication and mention of the grant of the patent: **14.08.1996**
(21) Application number: 92109410.8
(22) Date of filing: 03.06.1992
(51) Int. Cl.: C12P 35/06, C12N 9/78, C12N 1/16

(54) **Process for the enzymatic production of cephalosporane derivatives**
Verfahren zur enzymatischen Herstellung von Cephalosporinderivaten
Procédé pour la préparation enzymatique de dérivés de céphalosporine

(30) Priority: 03.06.1991 IT MI911509
(43) Date of publication of application: 09.12.1992
(73) Proprietor: MINISTERO DELL' UNIVERSITA' E DELLA RICERCA SCIENTIFICA E TECNOLOGICA, I-00196 Roma (IT)
(72) Inventor: Battistel, Ezio Angelo, I-19100 La Spezia (IT); Cesti, Pietro, I-28069 Trecate (Novara) (IT); Franzosi, Giuliana, I-20088 Cavignasco (IT); van der Goes, Vilhelmus, I-10014 Caluso (Torino) (IT); Bonicelli, Silvana, I-24010 Ponteranica (BG) (IT); Pilone, Mirella, I-20121 Milano (IT)
(74) Representative: Weinhold, Peter, Dr.

(56) References cited:
- EP-A- 0 474 211
- DE-A- 1 939 341
- FR-A- 2 133 927

## Description

The present invention relates to a process for the enzymatic preparation of cephalosporane derivatives of general formula (I): 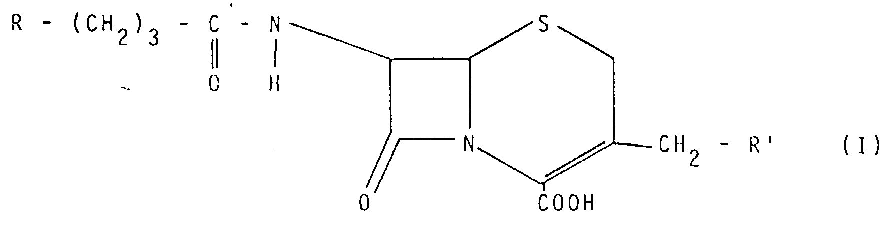 and their salts, wherein R represents a -CO-COOH or -COOH group and R' is H, OH or -O-CO-R", R" representing a C₁-C₄ alkyl group (e.g. methyl, ethyl, n- or i-propyl).

More specifically, the invention relates to an enzymatic process for the conversion of derivatives of cephalosporine C corresponding to general formula (II): 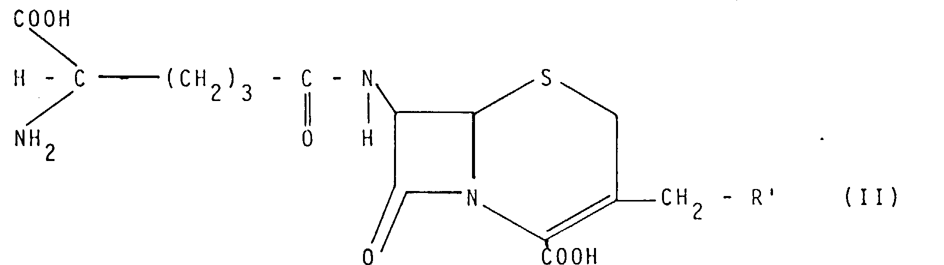 wherein R' has the above meaning, to compounds of formula (I), using an enzyme, in either free or immobilized form, which is capable of selectively oxidizing the amine group of the aminoadipic side chain of compounds of formula (II).

Cephalosporine C, i.e., 3-acetoxymethyl-7-(D-5-amino-5-carboxypentamide)ceph-3-em-4-carboxylic acid, can be converted to 3-acetoxymethyl-7-aminoceph-3-em-4-carboxylic acid (7-ACA) by removing the aminoadipic side chain of the β-lactame ring. 7-ACA is a compound of great industrial interest, in that it may be used in the synthesis of cephalosporane derivatives having antibacterial activity.

The deacylation of cephalosporine C, i.e., the elimination of the D-5-amino-5-carboxypentanoyl side chain, can be carried out chemically, for example, by using nitrosyl chloride in formic acid in the presence of acetonitrile (US-A-3,367,933). Another deacylation method involves the protection of the carboxyl group of the aminopentenoic side chain, reaction with phosphorus pentachloride at -55°C amd subsequent hydrolysis at low temperature with a mixture of water and methanol (BE-A-718,824).

These methods must generally be carried out at low temperatures and require the use of costly and toxic solvents or reagents, which can cause serious problems of pollution and environmental impact. In addition, owing to the chemical instability of the β-lactame ring and the reactivity of the groups present in the 3- and 7-positions of the ring, special reaction conditions must be applied (low temperatures, use of co-solvents) which make the process difficult to carry out on an industrial scale.

Enzymatic methods for removing the aminoadipic side chain in position 7 of the β-lactame ring of cephalosporine C are also known. For example, it is possible to use specific acylases (FR-A-2,241,557, US-A-4,774,179, EP-A-283,218) for selectively removing the D-aminoadipic side chain of cephalosporine C. These processes, however, are often not reproducible and are characterized by low yields and lengthy reaction times.

On the other hand, processes which transform the cephalosporine C into 7-ACA by means of two enzymatic stages are important from the industrial point of view. The first stage consists of using a D-amino acid oxidase, such as one derived from a microorganism of the Triagnopsis type (GB-A-1,272,769), which oxidizes the D-5-aminocarboxypentanoyl side chain in compounds of formula (I). In the second stage, an acylase is used, for example one of a Pseudomonas-type microorganism (US-A-3,960,662), which deacylates the compounds of formula (I) to the corresponding derivatives of 7-ACA.

These enzymatic processes are interesting from an industrial point of view in that they make it possible to eliminate the problems associated with the chemical synthesis with respect to the use and disposal of toxic compounds which are harmful to the environment.

These reactions are, in fact, carried out in an aqueous environment, under moderate conditions of pH and temperature, and do not involve the use of toxic substances or solvents.

The enzymes used in the first stage of these processes (D-amino acid oxidases) are, however, characterized by a catalytic activity which is not very stable under operating conditions and a sensitive lability of the bond between the prostetic part (cofactor) and the proteic part of the enzymatic molecule; see, e.g., Kubicek-Pranz, E.M. et al. (1985) J. Appl. Bioch. 7.104; and Szwajcer-Dey, E. et al. (1990) Biochemistry International 20, 1169.

It has now been found that it is possible to prepare compounds of formula (I) in a simple way, effectively and economically by means of a microbiological process which comprises the oxidative deamination of compounds of formula (II) by means of the enzyme D-amino acid oxidase derived from a microorganism of the species Rhodotorula glutinis in free or immobilized form. Particular advantages can be obtained by using as the enzymatic reagent the enzyme obtainable from Rhodotorula glutinis, deposited on 18/4/1991 at NCIMB (National Collections of Industrial & Marine Bacteria Ltd.) under the number 40412, one of its mutants or genetic material derived therefrom.

The present invention, consequently provides a process for the preparation of compounds of formula (I) which comprises the treatment of compounds of formula (II), preferably in an aqueous buffer solution, with the enzyme D-amino acid oxidase produced by a microorganism of the species Rhodotorula glutinis, one of its mutants or genetic material derived thereform which is capable of selectively oxidizing the D-amino acid of the side chain of the cephalosporine C in the 7-position to the corresponding acid or keto acid.

The process of the present invention is particularly effective in the preparation of the following derivatives encompassed by formula (I): 3-acetoxymethyl-7-β-(5-carboxy-5-oxopentanamide)-ceph-3-em-4-carboxylic acid and 3-acetoxymethyl-7-β-(4-carboxybutanamide)-ceph-3-em-4-carboxylic acid (glutaryl 7-ACA).

The microorganism, also subject of the present invention, has the following characteristics:
- Morphological characteristics: on agarized culture medium the colonies are pink/red in colour, have a mucoid consistency, shiny on the surface, with regular contours. The vegetative reproduction occurs by budding; the microscope does not reveal the presence of filaments. There is no sexual reproduction.
The micro-organism grows in 24-36 hours on YMG culture having the following composition:

| | |
|---|---|
| Yeast extract | 4 g/l |
| Malt extract | 8 g/l |
| Peptone | 5 g/l |
| Agar | 20 g/l |
| Glucose | 10 g/l |
| Growth temperature | 28-30°C |

- Biochemical characteristics: biochemical growth tests were carried out on different organic substrates, which are shown below together with the results obtained:

| | | | |
|---|---|---|---|
| Sorbitol | + | Galactose | + |
| D-Xylose | + | Actidione* | - |
| Ribose | + | Sucrose | + |
| Glycerol | + | N-acetyl-Glucosamine | - |
| Rhamnose | - | DL-lactate | - |
| Palatinose | + | L-Arabinose | + |
| Erythritol | - | Celbiose | + |
| Melbiose | - | Raffinoise | + |
| Glucuronate | - | Maltose | + |
| Melezitose | + | Trealose | + |
| Gluconate | - | 2-Keto-Gluconate | - |
| Levulinate | - | -Methylm-D-Glucoside | - |
| Glucose | + | Mannitol | + |
| Sorbose | - | Lactose | - |
| Glucosamine | - | Inositol | - |
| Esculine | - | | |

| | | | |
|---|---|---|---|
| * Actidione = Cycloheximide | | | |

On the basis of the physiological data the microorganism is to be considered a Rhodotorula glutinis, in accordance with the classification and characterization of this yeast given by Barnett et al. in "Yeasts: Characteristics and Identification", Cambridge University Press, 1990.

The microorganism preferably used in the process of the present invention may be grown on a standard culture medium using, for example, glucose as carbon source and peptone and yeast extract as nitrogen and vitamin source, at pH of 5 to 6. Under these conditions, the R. glutinis produces the enzyme D-amino acid oxidase, even though in low quantities. It has been found, however, that it is possible to increase the expression level of the enzyme of the microorganism by adding inducers to the culture medium. For this purpose, D-amino acids, such as D-alanine or D-methionine, may be used as sole nitrogen source, or, alternatively, the racemic mixtures thereof. These compounds may be added to the culture medium in concentrations ranging from 0.1 to 1.5%, preferably from 0.4 to 1% (w/v). As carbon source glucose may be used in a concentration of 0.1 to 2%, preferably 0.8 to 1.5% (w/v).

The oxidation reaction cannot be carried out with the whole cells, and consequently the D-amino acid oxidase contained in R. glutinis must be extracted or released from the cells before being used. For this purpose, the cellular wall and/or membrane can suitably be permeabilized, for example by using a solution of toluene and ethanol (2.5% v/v)at low temperature. Alternatively, it is preferable to use lysates or cellular extracts or enzymatic products with varying degrees of purification. For example, the cellular lysates may be prepared by suspending the cells in an aqueous buffer solution and breaking them both by means of vigorous stirring in the presence of glass pellets and by sonication with ultrasonic waves. The sample may subsequently be centrifuged to remove cellular deposits, whereas the D-amino acid oxidase activity remains in solution in the supernatant. A further concentration and purification of the cellular extract may be obtained, for example, by means of fractionation by precipitation with ammonium sulphate or by treatment with polyfunctional resins such as Whatman® CDR. A higher degree of purification may be obtained by weak ion-exchange resin column chromatography, hydrophobic interaction chromatography and by gel filtration.

The enzymatic activity of D-amino acid oxidase can conveniently be determined by measuring the consumption rate of the oxygen dissolved in water. This test can be carried out by polarography, using a specific electrode for the oxygen [Pilone-Simonetta, M. et al., Biochim. Biophys. Acta 914, 136-142 (1987)]. A D-amino acid, or its racemic mixture, such as alanine is added to the enzyme sample at a constant temperature of 37°C. More specifically, the reaction is carried out in a 100 mM pyrophosphate buffer, pH 8.5, and with 0.5% D-alanine as substrate, at 37°C.

The enzymatic activity (units per millilitre, U/ml, where one unit is the quantity of enzyme which converts 1 µmole of substrate per minute) is defined as follows:$\text{U/ml = v x α x (Vt/Vc)}$ wherein v is the initial consumption rate of the oxygen (in minutes), α is the solubility of the oxygen in water, Vt is the total volume of the test mixture and Vc is the sample volume.

Alternatively, the D-amino acid oxidase enzymatic activity can be determined by measuring the formation rate of hydrogen peroxide by spectrophotometry. In fact, the oxidative deamination reaction takes place with an accompanying formation of hydrogen peroxide which in turn can be titrated by reaction with 4-aminophenazone in the presence of dichlorophenyl sulphonate and peroxidase [P. Trinder et al., Ann. Clin. Biochem. 21, 430-433 (1984)].

The hydrogen peroxide which is formed during the reaction reacts with the peroxidase with the subsequent formation of a red-coloured quinonimine. More specifically, the reagent is prepared by dissolving dichlorophenyl sulphonate (1 g/l), 4-aminophenazone (0.4 g/l) and peroxidase (40 mg/l) in a 100 mM pyrophosphate buffer, pH 8.5. D-Alanine is added to the reagent as a substrate and also to the enzymatic sample. The units per millilitre, U/ml, are defined as follows:$\text{U/ml = (DO x Vt) / (19 x Vc)}$ wherein DO is the change of the optical density at 510 nm per minute, Vt is the total volume of the test mixture, 19 is the extinction coefficient of the quinonimine and Vc is the volume of the enzymatic sample.

The oxidation of the substrate of formula (II) is usually carried out in an aqueous buffer solution at pH 5 to 10, preferably 7 to 9, and at a temperature of 5 to 50°C, preferably 20 to 40°C. The substrate of formular (II) can generally be used up to a concentration of 20% (w/v), preferably 1 to 10%. The ratio substrate/enzyme usually ranges from 1 to 40, preferably from 10 to 30 mg/unit of enzyme. During the reaction air or oxygen is bubbled into the reaction mixture.

By treating compounds of formula (II) with D-amino acid oxidase in the presence of the enzyme catalase, compounds corresponding to formula (I) wherein R is -CO-COOH are obtained. This enzyme has the function of preventing the decarboxylation of the ketoacid owing to the hydrogen peroxide produced by the enzymatic deamination reaction, and consequently prevents the formation of the corresponding carboxylic acid. The catalase is normally present in the enzymatic complement of R. glutinis or in its cellular crude lysates.

To obtain a high yield in ketoacid it may be necessary, however, to add catalase to the reaction mixture. The amount of catalase to be added usually ranges from 0.01 to 0.5% (w/v). The catalase activity may be measured by means of spectrophotometry in accordance with the method described by Aebi, H. in "Methods in Enzymatic Analysis", Vol.2, 673-684, Bergmeyer, H.U., ed., Academic Press, N.Y. (1974). More specifically, hydrogen peroxide (concentration 30 mM) is added to the enzymatic test sample and the disappearance of the absorbance due to the presence of the peroxide, is observed, at 240 nm as a function of time, at 25°C.

Compounds of formula (I) wherein R is the -COOH group may be produced by treating compounds of formula (II) with D-amino acid oxidase as described above, without catalase activity, using sufficiently purified enzymatic preparations and carrying out the reaction in the presence of catalyse inhibitors. Examples of suitable inhibitors are ascorbic acid and sodium azide, which are generally used in concentrations of 1 to 100 mM.

The complete oxidation process may be carried out by vigorously stirring the mixture of the substrate of formula (II), the biocatalyst and the catalase (when ketoacids having formula (I) wherein R is CO-COOH are to be produced) in an aqueous buffer solution, by bubbling in air or oxygen.

The aeration of the mixture is important for obtaining an effective conversion. Generally aeration by bubbling in 1 volume of gas/system volume/minute is sufficient for obtaining a good yield.

The D-amino acid oxidase may be used in either free or immobilized form. The enzyme can appropriately be immobilized on either synthetic or natural polymer supports which are suitably functionalized to allow the covalent linkage of the proteic molecule. For example, agarose (Pharmacia), aminoalkylated glass with controlled porosity (EPC, 500 Å, Pierce), aminoalkylated silica (Fluka, 375 Å) and Duolite® (polystyrene polymer, Rohm and Haas) may be used for said purpose.

At the end of the reaction, the compound of formula (I) can be extracted from the solution, after acidification, with a suitable organic solvent such as n-butanol or ethyl acetate. Alternatively, the reaction product may be more conveniently extracted by using anion exchange resins, such as Amberlite® IRA 400 I, or absorbent resins, such as Amberlite® XAD-2. The product can be recovered by eluting the resin with a saline solution (for example carbonate) or with a suitable organic solvent (for example acetone or methanol). Alternatively, the reaction mixture may be directly used in the deacylation reaction for the production of 7-ACA.

### EXAMPLE 1

Cultures in liquid were prepared, in 500 ml flasks containing 100 ml of organic medium, in the presence of 0.2% of yeast extract, 0.5% of malt extract and 0.9% of D-alanine, pH 6. After 30 hours of fermentation (optical density at 660 nm of about 5), the cells where centrifuged and resuspended in a 100 mM phosphate buffer, pH 7.5, 5 mM mercaptoethanol and 2 mM EDTA, and collected for centrifugation after two washings. A cellular extract was prepared by subjecting the cells, suspended in the above buffer with the addition of 0.3% cetylpyridinium bromide, to mechanical breakage using glass beads (0.5 mm in diameter) under vigorous stirring, in the following proportion: 1 gram of cells/10 grams of beads/7 ml of buffer. Five breaking cycles (60**"**) were carried out. The homogenizate was filtered on a buchner, adding a few drops of 2-octanol to reduce the formation of foam, and was then centrifuged for 1 hour at 18,000 rpm. The supernatant was used for the reaction with cephalosporine C. 70 ml of 100 mM pyrophosphate buffer, pH 8, 100 mg of catalyse (Sigma, 2900 U/mg) and 1 g of cephalosporine C were added to 30 ml of supernatant (15 U/ml, specific activity 0.6 U/mg protein; the concentration of protein was estimated by means of the method of Bradford, M.H., Anal. Biochem. 72, 248-254 (1976)). The reaction temperature was controlled at 37°C, and air was bubbled into the mixture. An almost total disappearance of the substrate and also the formation of the deamination product, 3-acetoxymethyl-7-β-(5-carboxy-5-oxopentanamide)-ceph-3-em-4-carboxylic acid, was observed after about 60 minutes. The conversion into ketoacid was 93%, determined by HPLC (RP C18 Merck column (15x0.46 cm), eluant 25 mM phosphate buffer, pH 4.5, and acetonitrile, 91:9, flow rate 0.6 ml/min).

The solution was cooled to 0°C, saturated with ammonium sulphate, centrifuged, acidified to pH 3 with hydrochloric acid and extracted with ethyl acetate (4x100 ml).

The organic phase, washed with a saturated aqueous solution of ammonium sulphate, was dried over sodium sulphate and concentrated. The product was then crystallized from ethyl acetate/hexane. 400 mg of ketoacid were obtained (elemental analysis: C₁₆H₁₈N₂OS: calculated C 46.35%; H 4.4%; N 6.76%; S 7.75%; found C 46.12%; H 4.2%; N 6.85%; S 7.7%).

### EXAMPLE 2

The reaction was carried out under the same conditions as described in example 1, without addition of catalase, but in the presence of one of its inhibitors, i.e., sodium azide in a concentration of 50 mM. The reaction mixture was kept under aeration by bubbling in air and at a thermostated temperature of 37°C. After about 60 minutes the cephalosporine C had almost completely reacted and the reaction product, i.e., 3-acetoxy-methyl-7-β-(4-carboxybutanamide)-ceph-3-em-4-carboxylic acid (glutaryl-7-ACA), had formed. The conversion to glutaryl-7-ACA was 91%, determined by means of HPLC, as described above.

The reaction mixture was treated with the absorbent resin Amberlite®XAD-2 (20 g). The resin was subsequently filtered, washed with water and then with methanol (200 ml). The organic phase was concentrated under vacuum and the product crystallized from ethyl acetate/hexane. 440 mg of glutaryl 7-ACA were obtained. Elemental analysis: C₁₅H₁₈N₂O₈S: calculated C 46.63%; H 4.70%; N 7.25%; S 8.30%; found: C 46.41%; H 4.62%; N 7.45%; S 8.38%.

### EXAMPLE 3

Ammonium sulphate (30% saturation at 4°C) was added to the cellular lysate (200 ml) prepared as described in example 1 (2 U/ml, 0.3 U/mg protein). The solution was left to stand for 1 hour and was then centrifuged at 10,000 rpm for 30 minutes. Ammonium sulphate (60% saturation) was added to the supernatant. The suspension was left to stand for 1 hour and was then centrifuged at 15,000 rpm for 30 minutes. The sediment, resuspended in 5 ml of 10 mM potassium pyrophosphate buffer, pH 7.5, EDTA (2 mM), glycerol (15%) and 5 mM mercaptoethanol, was dialyzed against the same buffer. 7 ml of enzymatic solution (50 U/ml, 0.4 U/mg) were thus obtained. The enzyme was further purified by chromatography on a DEAE-Sephacel®column equilibrated with the same buffer. The fractions not withheld and eluted from the column were concentrated to obtain 10 ml of enzymatic solution (20 U/ml, 12 U/mg). The D-amino acid oxidase, partially purified as described above, was used for the reaction with cephalosporine C. 7.5 ml of 100 mM pyrophosphate buffer, pH 8, and 100 mg of cephalosporine C were added to 2.5 ml of this solution. The temperature of the mixture was controlled at 37°C for about 90 minutes, and air was bubbled in. Thereby an almost total conversion of cephalosporine C into 3-acetoxymethyl-7-β-(4-carboxybutanamide) -ceph-3-em-4-carboxylic acid (glutaryl-7-ACA) was obtained. The conversion was equal to 91% in glutaryl-7-ACA, determined by HPLC under the conditions described above.

## Claims

1. Process for the enzymatic preparation of cephalosporane derivatives having general formula (I): 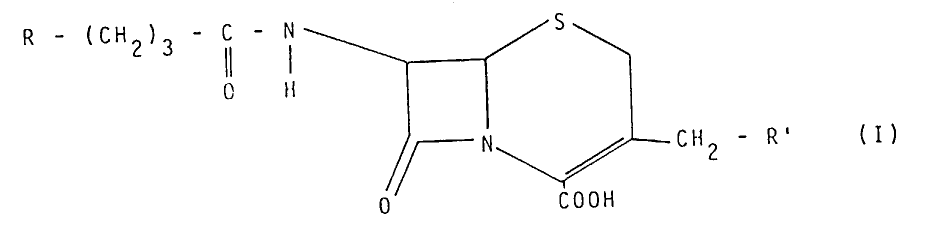 wherein R represents -CO-COOH or -COOH and R' is H, OH or -O-CO-R", R" being C₁-C₄ alkyl; or salts thereof, said process comprising the oxidative deamination of derivatives of cephalosporine C corresponding to general formula (II): 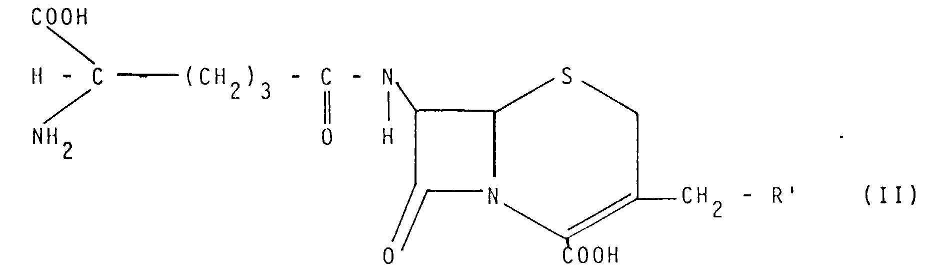 wherein R' is as defined above; or salts thereof, in the presence of the enzyme D-amino acid oxidase capable of said deamination, in free or immobilized form, obtainable from a microorganism of the species Rhodotorula glutinis.

2. Process according to claim 1, wherein the microorganism is Rhodotorula glutinis NCIMB No. 40412 and/or a mutant thereof capable of producing said D-amino acid oxidase.

3. Process according to any one of claims 1 and 2, wherein the reaction is carried out in an aqueous buffer solution, particularly a buffer solution of a pH of from 5 to 10, preferably 7 to 9.

4. Process according to any one of claims 1 to 3, wherein the reaction is carried out at a temperature of from 5 to 50°C, preferably 20 to 40°C.

5. Process according to any one of claims 1 to 4, wherein the reaction is carried out at a concentration of the substrate of formula (II) of up to 20% w/v, preferably from 1 to 10% w/v.

6. Process according to any one of claims 1 to 5, wherein the reaction is carried out at a substrate/enzyme ratio of from 1 to 40, preferably 10 to 30 mg/unit of enzyme.

7. Process according to any one of claims 1 to 6, wherein the reaction is carried out in the presence of a catalase added to the mixture, preferably in amounts of from 0.01 to 0.5% (w/v).

8. Process according to any one of claims 1 to 6, wherein the reaction is carried out in the presence of an inhibitor of catalase, particularly a catalase inhibitor selected from ascorbic acid and sodium azide, preferably in a concentration thereof ranging from 1 to 100 nM.

9. Microorganism Rhodotorula glutinis NCIMB No. 40412.

10. D-Amino acid oxidase capable of deaminating cephalosphorine C derivatives of general formula (II) and obtainable from the microorganism Rhodotorula glutinis NCIMB No. 40412 or mutants thereof.

## Patentansprüche

1. Verfahren zur enzymatischen Herstellung von Cephalosporan-Derivaten der allgemeinen Formel (I): 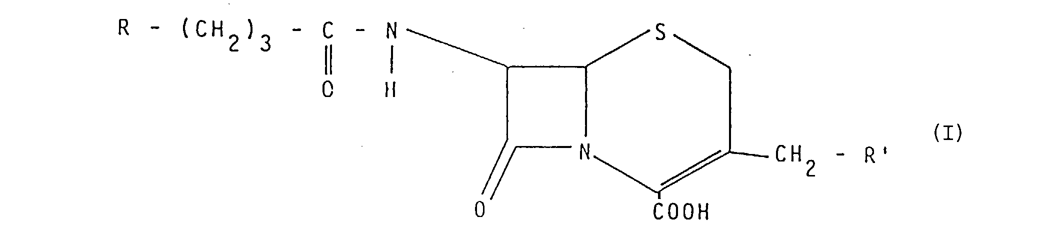 worin R -CO-COOH oder -COOH bedeutet und R' H, OH oder -O-CO-R" mit R" in der Bedeutung C₁-C₄-Alkyl ist, oder von dessen Salzen, umfassend die oxidative Desaminierung von Cephalosporin C-Derivaten der allgemeinen Formel (II): 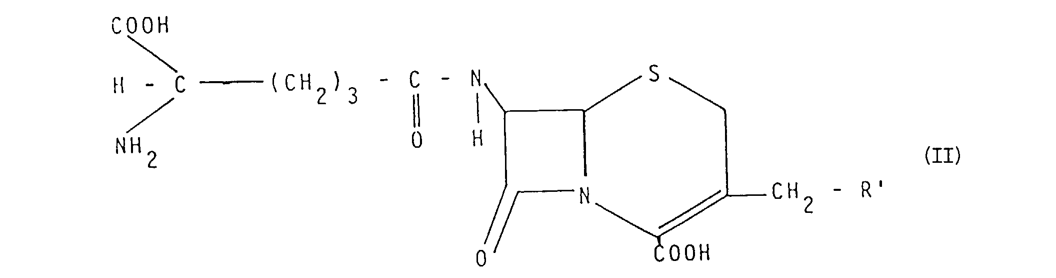 worin R' die obige Bedeutung hat, oder von dessen Salzen in Gegenwart, in freier oder immobilisierter Form, des zu dieser Desaminierung fähigen und aus einem Mikroorganismus der Spezies Rhodotorula glutinis erhältlichen Enzyms D-Aminosäure-Oxidase.

2. Verfahren nach Anspruch 1, worin der Mikroorganismus Rhodotorula glutinis NCIMB Nr. 40412 und/oder eine seiner Mutanten ist, die zur Produktion der D-Aminosäure-Oxidase fähig ist.

3. Verfahren nach einem der Ansprüche 1 und 2, worin die Umsetzung in einer wäßrigen Pufferlösung durchgeführt wird, insbesondere einer Pufferlösung mit einem pH von 5 bis 10, vorzugsweise von 7 bis 9.

4. Verfahren nach einem der Ansprüche 1 bis 3, worin die Umsetzung bei einer Temperatur von 5 bis 50°C, vorzugsweise von 20 bis 40°C durchgeführt wird.

5. Verfahren nach einem der Ansprüche 1 bis 4, worin die Umsetzung bei einer Konzentration an Substrat der Formel (II) von bis zu 20% w/v, vorzugsweise von 1 bis 10% w/v, durchgeführt wird.

6. Verfahren nach einem der Ansprüche 1 bis 5, worin die Umsetzung bei einem Substrat/Enzym-Verhältnis von 1 bis 40, vorzugsweise von 10 bis 30 mg/Enzymeinheit durchgeführt wird.

7. Verfahren nach einem der Ansprüche 1 bis 6, worin die Umsetzung in Gegenwart einer Katalase durchgeführt wird, die der Mischung vorzugsweise in einer Menge von 0,01 bis 0,5% (w/v) zugefügt ist.

8. Verfahren nach einem der Ansprüche 1 bis 6, worin die Umsetzung in Gegenwart eines Katalaseinhibitors, insbesondere eines Katalaseinhibitors, der aus Ascorbinsäure und Natriumazid ausgewählt ist, durchgeführt wird, dessen Konzentration vorzugsweise zwischen 1 und 100 nM liegt.

9. Mikroorganismus Rhodotorula glutinis NCIMB Nr. 40412.

10. D-Aminosäure-Oxidase, die zur Desaminierung von Cephalosporin C-Derivaten der allgemeinen Formel (II) fähig ist und aus dem Mikroorganismus Rhodotorula glutinis NCIMB Nr. 40412 oder dessen Mutanten erhältlich ist.

## Revendications

1. Procédé de préparation par voie enzymatique de dérivés de la céphalosporine de formule générale (I) 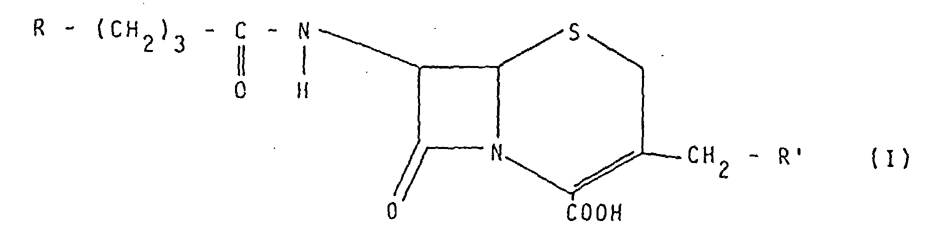
dans laquelle R représente un groupe -CO-COOH ou -COOH et R' est un atome d'hydrogène, un groupe OH ou -O-CO-R", R" représentant un groupe alkyle en C₁₋₄,
ou de sels de tels composés,
ledit procédé comprenant la désamination par voie oxydante de dérivés de la céphalosporine C correspondant à la formule générale (II) 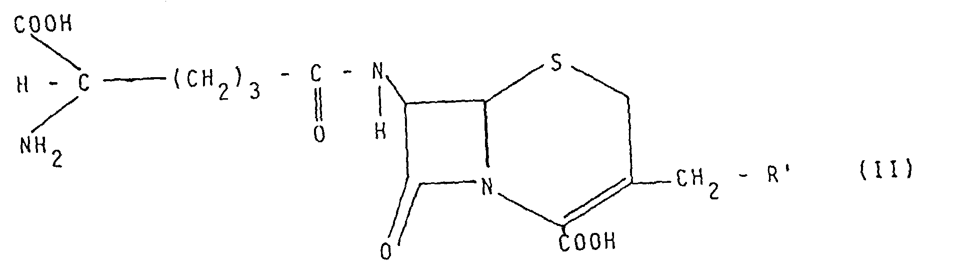 dans laquelle R' est défini ci-dessus, ou de sels de tels composés, en présence de l'enzyme D-amino-acide oxydase capable d'effectuer ladite désamination, sous forme libre ou immobilisée, que l'on peut obtenir à partir d'un microorganisme de l'espèce *Rhodotorula glutinis.*

2. Procédé conforme à la revendication 1 dans lequel le microorganisme est *Rhodotorula glutinis* NCIMB n° 40412 et/ou un mutant de ce microorganisme, capable de produire ladite D-amino-acide oxydase.

3. Procédé conforme à une quelconque des revendication 1 et 2, dans lequel la réaction est effectuée dans une solution tampon aqueuse en particulier dans une solution tampon ayant un pH compris entre 5 et 10, de préférence entre 7 et 9.

4. Procédé conforme à une quelconque des revendications 1 à 3, dans lequel la réaction est effectuée à une température comprise entre 5 et 50 °C, de préférence entre 20 et 40 °C.

5. Procédé conforme à une quelconque des revendications 1 à 4, dans lequel la réaction est effectuée à une concentration en substrat de formule (II) allant jusqu'à 20 % en poids/volume, de préférence comprise entre 1 et 10 % en poids/volume.

6. Procédé conforme à une quelconque des revendications 1 à 5, dans lequel la réaction est réalisée avec un rapport substrat/enzyme compris entre 1 et 40, de préférence entre 10 et 30 mg/unité d'enzyme.

7. Procédé conforme à une quelconque des revendications 1 à 6, dans lequel la réaction est réalisée en présence d'une catalase ajoutée au mélange, de préférence à raison de 0,01 à 0,5 % (poids/volume).

8. Procédé conforme à une quelconque des revendications 1 à 6, dans lequel la réaction est effectuée en présence d'un inhibiteur de la catalase, en particulier un inhibiteur de la catalase choisi parmi l'acide ascorbique et l'azide de sodium, de préférence à une concentration comprise entre 1 et 100 nM.

9. Microorganisme *Rhodotorula glutinis* NCIMB n° 40412.

10. D-amino-acide oxydase capable d'effectuer la désamination de dérivés de la céphalosporine C de formule générale (II) et que l'on peut obtenir à partir du microorganisme *Rhodotorula glutinis* NCIMB n° 40412 ou de mutants de ce microorganisme.
